# EUROPEAN PATENT SPECIFICATION

(11) **EP 2 902 073 B1**
(45) Date of publication and mention of the grant of the patent: **12.06.2019**
(21) Application number: 12885773.7
(22) Date of filing: 28.09.2012
(51) Int. Cl.: A61M 39/02, F16K 15/18, F16L 37/40, A61M 39/22

(54) **CONNECTOR**
VERBINDER
CONNECTEUR

(43) Date of publication of application: 05.08.2015
(73) Proprietor: Terumo Kabushiki Kaisha, Tokyo 151-0072 (JP)
(72) Inventor: UEDA, Yasuhiro, Nakakoma-gun Yamanashi 409-3853 (JP); HAMA, Yoshikazu, Tottori-shi, Tottori 680-0945 (JP)
(74) Representative: Casalonga
(86) International application number: PCT/JP2012/075016
(87) International publication number: WO 2014/049811

(56) References cited:
- EP-A1- 0 499 401
- WO-A2-2008/091698
- JP-A- 2002 306 610
- JP-A- 2003 144 546
- JP-A- 2004 105 574
- JP-B2- 3 389 983
- JP-B2- 4 744 440
- US-A- 5 549 651
- US-A1- 2010 007 134

## Description

### Technical Field

The present invention relates to a connector that allows and inhibits circulation of fluid by opening and closing a slit of a valve.

### Background Art

Conventionally, when an intravenous fluid is administered to a patient, multiple tubes are connected to form an infusion line from an infusion bag to the patient, wherein the multiple tubes are connected with one another with a connector. Upon an administration of an intravenous fluid, there may be the case in which other transfusion material is supplied to a main line, which is for supplying a main transfusion material to a patient, from another line, and they are mixed to be supplied to the patient. In this case, a three-port connector into which multiple types of transfusion materials can be mixed is used for the connector (see JP 3389983 B1).

The connector (medical mixing injection port) described in JP 3389983 B1 includes a connection port to which a male connector (inserted body) of another line is connected in addition to two ports forming a main line. This connection port is formed with a valve having a slit that is opened and closed upon an insertion and removal of the male connector to allow or inhibit circulation of fluid. When the male connector is connected to the connector, the valve is displaced toward a body by the male connector to open the slit according to the elastic deformation of the valve. Thus, the male connector penetrates through the valve, whereby communication between the main line and another line is allowed.

However, when a connection structure in which the male connector penetrates through the slit as described above is employed, the slit of the valve is deformed so as to be pushed and opened by the male connector. Therefore, a periphery of the slit of the valve is relatively easily broken. When the valve is broken as described above, the closing force of the valve is reduced, which might entail a problem of a high possibility of a leakage of fluid. Further prior art connectors are disclosed in WO 2008/091698 A2, US 5,549,651 and EP 0 499 401 A1.

### Summary of Invention

The present invention as defined in claim 1, is accomplished in view of the above circumstance, and aims to provide a connector that can prevent a penetration of an inserted body through a slit of a valve with a simple structure, resulting in being capable of reducing damage of the valve, and being satisfactorily used multiple times or over a long period of time.

In order to attain the forgoing object, the present invention provides a connector including a body including a flow channel through which fluid can be circulated, and a space communicating with the flow channel; and a valve including a base disposed in the space so as to be displaceable, and a slit that can open and close the flow channel based on elastic deformation of the base, wherein the body has a stopper provided on a way, on which the valve is displaced, for restricting a predetermined or more degree of elastic deformation of the valve.

According to the configuration described above, the body of the connector has the stopper on the way on which the valve is displaced. Therefore, even if the valve is deformed by an inserted body when the inserted body such as a male connector is connected to the connector, the stopper can restrict a predetermined or more degree of elastic deformation of the valve. The valve of which elastic deformation is restricted effectively prevents the inserted body from penetrating through the slit. As a result, the connector can reduce the situation in which the valve is broken by the inserted body that pushes and opens the slit. With this, the connector can satisfactorily be used multiple times or for a long period of time.

In this case, the stopper may hold the elastically deformed base with the inserted body when the inserted body is inserted into the body.

As described above, in the connector, the elastically deformed valve is sandwiched between the stopper and the inserted body in the state in which the inserted body is inserted, whereby the connector can easily specify the insertion length of the inserted body. Since the inserted body holds the valve upon the insertion, the insertion is restricted. This can surely prevent the inserted body from penetrating through the slit. In addition, the valve can contact the inserted body so as to cover the inserted body in an airtight or liquid-tight manner, thereby being capable of fixedly holding the inserted body.

In this case, the stopper may be formed from a wall face at a side part of the flow channel that is narrower than the space.

Since the stopper is formed from a wall face at the side part of the flow channel, the elastic deformation of the valve can easily be restricted without providing another member as the stopper, whereby production cost can be reduced.

In addition, the body preferably includes a housing that forms the flow channel, and a cover member that is mounted to the housing to form the space, and the stopper is preferably formed to be communicated with an exposure opening of the housing from which the flow channel is exposed.

Since the stopper is formed so as to be communicated with the exposure opening of the housing from which the flow channel is exposed, the connector can restrict the elastic deformation of the valve in the vicinity of the exposure opening. Therefore, the connector can allow the inserted body to be fully inserted to the vicinity of the exposure opening (flow channel) upon connection, and can satisfactory maintain the connection state with the inserted body.

In addition, the stopper is preferably formed to have a taper portion that becomes narrower toward the flow channel from a position where the valve is disposed.

Since the stopper is formed to have a taper portion, the connector can obliquely receive the valve that is elastically deformed due to the pushed inserted body at the taper portion. Accordingly, the connector can significantly reduce damage of the valve.

### Brief Description of Drawings

Fig. 1 is an explanatory view schematically illustrating one example of an infusion set to which a connector according to the present embodiment is applied.
Fig. 2 is a perspective view illustrating an entire configuration of the connector in Fig. 1.
Fig. 3A is a plan view illustrating a housing of the connector in Fig. 2, and Fig. 3B is a sectional view taken along a line IIIB - IIIB in Fig. 3A.
Fig. 4 is an explanatory view illustrating a state in which a third port is mounted to the housing in Fig. 3B.
Fig. 5 is an explanatory view illustrating a state in which a male connector is connected to the connector in Fig. 4.
Fig. 6 is a side sectional view illustrating an entire configuration of a connector according to a first modification.
Fig. 7A is a perspective view illustrating an entire configuration of a connector according to a second modification, and Fig. 7B is a perspective view illustrating a housing of the connector in Fig. 7A.
Fig. 8A is a perspective view illustrating an entire configuration of a connector according to a third modification, and Fig. 8B is a perspective view illustrating a housing of the connector in Fig. 8A.

### Description of Embodiments

A connector according to the present invention will be described in detail below based on a relationship with an infusion set to which the connector can be applied. It is obvious that the connector is not limited to be applied to an infusion set.

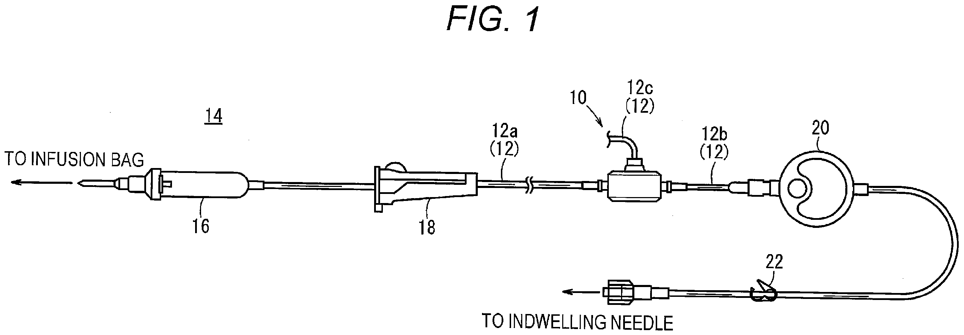

As stated previously, a connector 10 has a function of connecting multiple tubes 12 in an infusion line for administering an intravenous fluid to a patient, and is applied to an infusion set 14 as illustrated in Fig. 1, for example. The upstream side of the infusion set 14 is connected to an infusion bag not illustrated, while the downstream side is connected to an indwelling needle not illustrated, whereby the infusion set 14 forms an infusion line for administering a transfusion material to a patient from the infusion bag.

The transfusion material flowing through the infusion set 14 includes any fluids that can be administered to a living body, such as medical solution, correction electrolyte fluid, or normal saline solution. When the transfusion material is medical solution, various types of medical solutions can be selected, such as sedative, intravenous anesthetic, anesthetic sedative, local anesthetic solution, non-depolarizing muscle relaxant, pressor agent, depressor agent, coronary dilatation agent, diuretic agent, anti-arrhythmic agent, bronchodilating agent, hemostatic agent, vitamin preparation, antibiotic, and fat emulsion.

A tube 12 of the infusion set 14 includes, for example, a drip tube 16 by which a flow rate of a transfusion material supplied from the infusion bag can be visually confirmed, a clamp 18 for adjusting the flow rate of the transfusion material, an air vent filter 20 for exhausting (or supplying) air present in the infusion line, and a clamp 22 that closes the tube 12. It is obvious that the structure of the infusion set 14 is not limited to the one illustrated in Fig. 1. In addition to the above components, the infusion set can include various components (e.g., an infusion pump or a check valve) mounted on the infusion line.

The tube 12 in the infusion set 14 is a tube body having flexibility, and forms a flow channel of the transfusion material. When being applied to the above infusion set 14, the connector 10 is provided between the clamp 18 and the air vent filter 20, for example. Specifically, the connector 10 connects a first tube 12a extending from the drip tube 16 to the downstream side and a second tube 12b extending at the upstream side of the air vent filter 20 such that the transfusion material can be circulated between the first tube 12a and the second tube 12b. The connector 10 is a three-port connector by which a third tube 12c that is formed as another line can be connected to a main line composed of the first tube 12a and the second tube 12b.

The arrangement position of the connector 10 is not limited to the above position. The connector 10 can be provided at any desired position of the infusion set 14. In addition, the infusion set 14 (infusion line) may include one or more connectors 10.

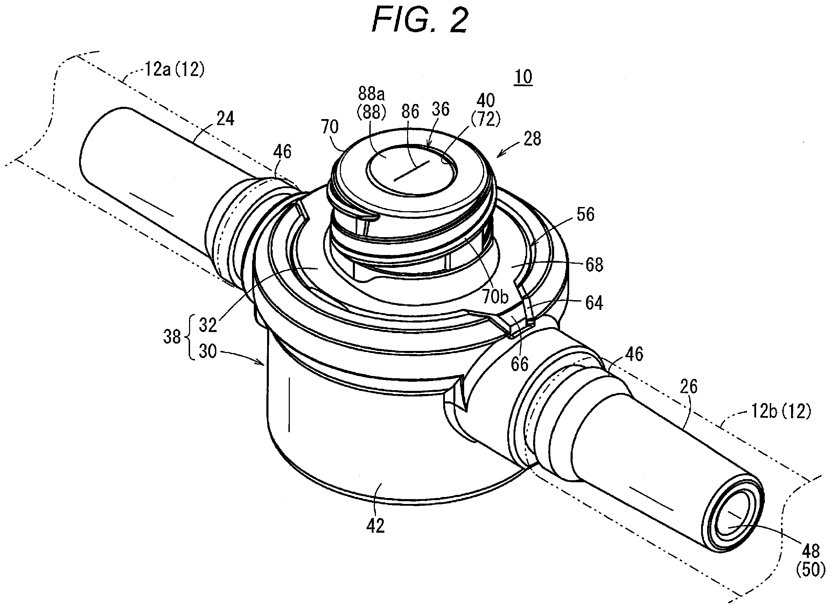

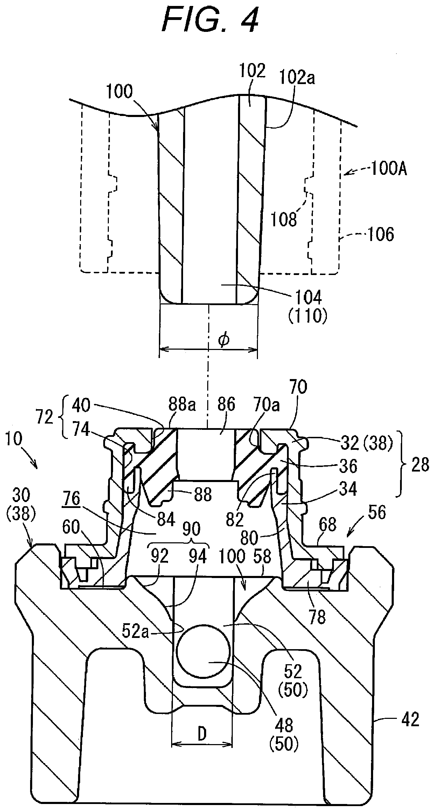

A specific structure of the connector 10 according to the present embodiment will be described in detail below. As illustrated in Fig. 2, the connector 10 includes a first port 24 to which the first tube 12a forming the main line is connected, a second port 26 to which the second tube 12b similarly forming the main line is connected, and a third port 28 to which the third tube 12c forming another line is connected. The first and second ports 24 and 26 are provided to a housing 30 (casing) having a flow channel of the transfusion material inside. The third port 28 is composed of a cap 32 (cover member), a support member 34 (see Fig. 4), and a valve 36, which are separate members from the housing 30. Each member is assembled to the housing 30 to form the third port 28.

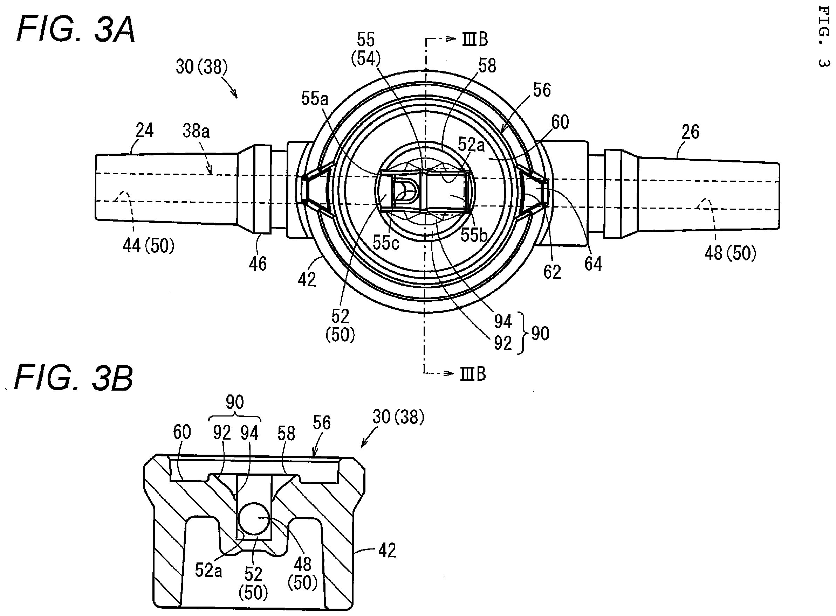

The housing 30 and the cap 32 are connected to each other to form a body 38 of the connector 10 in which a flow channel 38a (see Figs. 3A and 4) for a transfusion material is formed. The support member 34 and the valve 36 are stored in the body 38, and only the top surface of the valve 36 is exposed from an opening 40 of the cap 32.

The housing 30, the cap 32, and the support member 34 of the connector 10 are formed from a resin material in the light of easiness in forming process and reduced cost. A material having higher rigidity than the tube 12 is preferably used as the resin material. Examples of the resin material include polyethylene, polypropylene, polyolefin such as ethylene-vinyl acetate copolymer, polyurethane, polyamide, polyester, polycarbonate, polybutadiene, and polyvinyl chloride.

As illustrated in Figs. 2, 3A, and 3B, the housing 30 has a bottomed cylindrical connector base 42 at its center, and the first port 24 and the second port 26 are connected to the side peripheral face of the connector base 42. The first port 24 is formed to have a generally cylindrical shape, and linearly extends toward the upstream side of the main line from the connector base 42. A first port flow channel 44 through which the transfusion material can be flown is formed in the first port 24 to extend along the axial direction.

The first port 24 is formed as a male luer taper of which diameter is gradually decreased toward an extended end, and is inserted into a tube (internal cavity) of the first tube 12a. A protrusion 46 is formed along the circumferential direction on the outer peripheral surface of the first port 24 near the connector base 42. When the first tube 12a is attached beyond the protrusion 46, a liquid-tight connection is achieved between the first tube 12a and the first port 24.

On the other hand, the second port 26 is formed to have the same shape as the first port 24 at the opposite side of the first port 24 across the connector base 42, and linearly extends toward the downstream side of the main line from the connector base 42. A second port flow channel 48 through which the transfusion material can be flown is formed in the second port 26 to extend along the axial direction. Specifically, the first and second ports 24 and 26 are linearly formed in a line with the same axis, resulting in that the first and second port flow channels 44 and 48 are linearly communicated with each other. The connector 10 has these first and second port flow channels 44 and 48 linearly communicated with each other as a main line flow channel (hereinafter referred to as a main line flow channel 50). The connector 10 can allow a first transfusion material flowing through the main line flow channel 50 to smoothly flow.

The bottomed cylindrical connector base 42 has a thickness on its side peripheral face in order to connect and support the first and second ports 24 and 26. An internal flow channel 52 communicated with the first port flow channel 44 and the second port flow channel 48 is formed in the connector base 42. The internal flow channel 52 is a part of the main line flow channel 50, and is formed to linearly form the main line flow channel 50. A guide wall 54 that guides the first transfusion material to flow in the vertical direction (in the direction in which the cap 32 is connected) is formed at the center of the internal flow channel 52 in the axial direction.

The guide wall 54 has an expanding portion 55 that is formed with a large thickness close to the first port 24 from the center of the internal flow channel 52 in the axial direction. An upstream wall face 55a is formed on the expanding portion 55 at the position close to the first port flow channel 44, the upstream wall face 55a being opposite to the first port flow channel 44 and orthogonal to the first port flow channel 44. A downstream wall face 55b that gently tilts compared to the upstream wall face 55a is formed close to the second port flow channel 48. A cutout groove 55c formed by cutting the center in the width direction is formed on the upstream wall face 55a. The guide wall 54 guides the first transfusion material, which is flown from the first port flow channel 44, upward (toward the cap 32) by the upstream wall face 55a and the cutout groove 55c. With this, in a state in which the third tube 12c is not connected to the third port 28 (in a closing state of the valve 36), for example, the first transfusion material is guided to rush into a space (through-hole 76) of the third port 28, and can be flown toward the downstream side without being stayed in the space.

On the other hand, the top of the connector base 42 is formed as a mounting portion 56 for mounting the cap 32 and the support member 34. The mounting portion 56 is formed by cutting the top surface of the connector base 42 in a circular shape, and includes a circular exposure opening 58 from which the internal flow channel 52 is exposed at its center, an arrangement portion 60 that is formed flat to enclose the exposure opening 58, and hook walls 62 and grooves 64 that are formed outside the arrangement portion 60 and on base parts of the first and second ports 24 and 26. The support member 34 is arranged on the arrangement portion 60, and a locking claw 66 of the cap 32 is locked to the hook wall 62.

The diameter of the exposure opening 58 is formed larger than the width of the internal flow channel 52, and the internal flow channel 52 extends to cut out the center of the exposure opening 58 in a groove shape. A wall face is formed toward the edge of the exposure opening 58 at the side part continuous with a pair of side walls 52a forming the internal flow channel 52. This wall face functions as a stopper 90 for restricting a predetermined or more degree of elastic deformation of the valve 36. The stopper 90 will be described in detail later.

As described above, the third port 28 is a connection terminal including the cap 32, the support member 34, and the valve 36. The third port 28 is fixed to the mounting portion 56 in the direction orthogonal to the axial direction of the first and second ports 24 and 26. Specifically, the connector 10 is formed as a T-port connector in which the third port 28 has a branch angle of 90 degrees to the main line flow channel 50. A male connector 100 (inserted body) of the third tube 12c is connected to the third port 28.

As illustrated in Figs. 2 and 4, the cap 32 stores the valve 36 inside, and is formed to have an outer shape connectable to the male connector 100 of the third tube 12c. The cap 32 includes a lower flange 68 connected to the connector base 42, and a terminal portion 70 extending upward from the flange 68 with a predetermined length.

The flange 68 is formed to have an outer diameter covering the arrangement portion 60 of the connector base 42. A pair of locking claws 66 projecting toward the outside in the diameter direction is consecutively formed on the flange 68 at predetermined symmetric positions on its peripheral edge across the terminal portion 70. The pair of locking claws 66 is formed to have a hook shape, and the distance between the pair of locking claws 66 is slightly shorter than that of the pair of hook walls 62. The pair of locking claws 66 is fitted to the grooves 64 of the connector base 42, thereby being hooked to the hook walls 62.

The terminal portion 70 is formed to have a cylindrical shape with a diameter smaller than that of the flange 68, and has a hole 72 inside along an axial direction (vertical direction). The opening 40 of which diameter is smaller in the inner radial direction is formed at the upper part of the hole 72, and a storage portion 74 with a diameter larger than the opening 40 is formed below the opening 40, the storage portion 74 being capable of storing the support member 34 and the valve 36.

The opening 40 is enclosed by an annular projection 70a projecting downward from a top edge of the terminal portion 70, whereby the opening 40 is formed to have a predetermined internal diameter (an internal diameter by which the valve 36 can be inserted). The internal diameter of the opening 40 is set to be about 4 mm, for example, considering a handling property of the connector 10. A helical projection 70b to which the luer lock male connector is threaded is formed on the outer peripheral surface of the terminal portion 70.

Here, the male connector 100 of the third tube 12c connected to the connector 10 as another line of the infusion set 14 will be described with reference to Fig. 4. A luer slip male connector specified in the ISO standard is used as the male connector 100, for example. Specifically, the male connector 100 has an insertion cylinder 102 inserted into the connector 10 (cap 32).

The insertion cylinder 102 linearly extends in the axial direction, and upon the connection with the connector 10, the tip end (lower end in Fig. 4) of the insertion cylinder 102 pushes to open the slit 86 of the valve 36. A flow path 104 through which a second transfusion material supplied from the third tube 12c can be flown is formed in the insertion cylinder 102. Specifically, the flow path 104 is formed as a flow channel of another line (hereinafter referred to as an another line flow channel 110).

The outer peripheral surface of the insertion cylinder 102 has a taper surface 102a reducing diameter toward the tip end. Specifically, the insertion cylinder 102 is configured such that an outer diameter of a body that is inserted into the connector 10 with a predetermined length is the same as the internal diameter (e.g., 4 mm) of the opening 40 of the cap 32, and the outer diameter of the tip end is slightly smaller than the outer diameter of the body. Since the taper surface 102a is fitted to the opening 40 upon the insertion into the connector 10, the insertion cylinder 102 is connected to the third port 28 (the cap 32, the support member 34, and the valve 36).

It is obvious that the male connector connected to the third port 28 is not limited to the above luer slip male connector 100. For example, as indicated by a broken line in Fig. 4, a luer lock male connector 100A that includes a connection cylinder 106 enclosing the insertion cylinder 102 and a helical protrusion 108, which is formed on an inner peripheral surface of the connection cylinder 106 and threaded to the projection 70b of the cap 32, may be used.

On the other hand, the support member 34 of the third port 28 has a function of supporting the valve 36 at a position apart from the internal flow channel 52 with a predetermined space. Specifically, when the third port 28 and the male connector 100 are connected to each other, a space for inserting the male connector 100 (insertion cylinder 102) with a predetermined or more length is needed. The support member 34 supports the valve 36 at its top part, and further, forms the space by the through-hole 76 extending in the vertical direction.

The support member 34 has a flange portion 78 arranged on the arrangement portion 60 of the connector base 42, and a support cylinder 80 projecting upward from the top surface of the flange portion 78. The upper part of the support cylinder 80 is formed as a holding portion 82 with a diameter smaller than a diameter of a body part, and a fixing portion 84 of the valve 36 is fitted to the outside of the holding portion 82.

The through-hole 76 is formed to penetrate through the flange portion 78 and the support cylinder 80. The through-hole 76 is communicated with the exposure opening 58 with the support member 34 arranged on the arrangement portion 60. The through-hole 76 is formed as a flow channel in the third port 28 to allow the second transfusion material to flow through the main line flow channel 50 from the another line flow channel 110. Specifically, the flow channel 38a in the body 38 is composed of the first and second port flow channels 44 and 48, the internal flow channel 52, and the through-hole 76. When the insertion cylinder 102 is inserted, the insertion cylinder 102 and the elastically deformed valve 36 can move in the through-hole 76. The through-hole 76 and the opening 40 are formed as a space that allows the deformation of the valve 36.

Different from the other members, the valve 36 of the third port 28 is made of an elastic material, so that the valve 36 has elastic force by which the valve 36 is elastically deformed upon the insertion of the male connector 100. An elastic material of the valve 36 is not particularly limited. Examples of the elastic material include a synthetic rubber such as polybutadiene rubber, nitrile rubber, or chloroprene rubber; a natural rubber such as polyisoprene rubber; and thermoset elastomer, thermoplastic elastomer, or other elastomers such as urethane rubber, silicon rubber, or fluorine-contained rubber.

The valve 36 has the slit 86, which is opened and closed upon the insertion and removal of the male connector 100, at its center. The valve 36 has a function of allowing and inhibiting communication with the another line flow channel 110 by opening and closing this slit 86. The valve 36 is formed to have a disk-like shape with relatively a large thickness, and includes an outer fixing portion 84 held by the cap 32 and the support member 34, and an inner deformed portion 88 formed continuous with the fixing portion 84. The deformed portion 88 has an upper expanding portion 88a inserted into the opening 40. The outer diameter of the upper expanding portion 88a is set to be about 4 mm, for example, according to the inner diameter of the opening 40.

The slit 86 is formed to extend through the deformed portion 88 in the vertical direction, and is in a closing state when stored in the opening 40 (when the slit 86 is not elastically deformed). Depending on the size of the deformed portion 88, the length of the slit 86 in the longitudinal direction is set to be about 2 mm, for example. Upon the insertion of the male connector 100, the deformed portion 88 is displaced (elastically deformed) relative to the fixing portion 84, whereby the slit 86 is gradually opened.

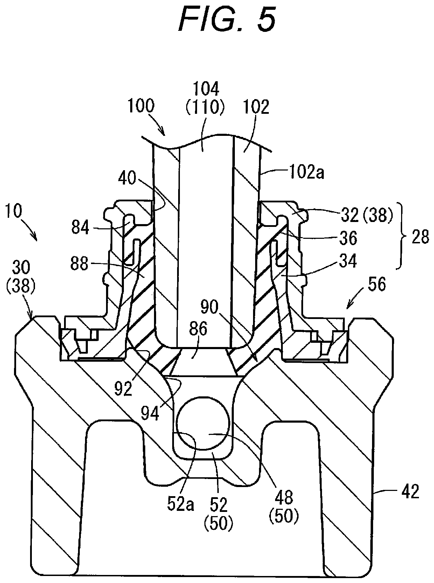

The elastic deformation of the deformed portion 88 is restricted by the stopper 90 at the position where the insertion cylinder 102 is inserted into the third port 28 with a predetermined length (see also Fig. 5). The configuration of the stopper 90 will be described below.

As illustrated in Figs. 3A, 3B, and 4, the stopper 90 is formed in the exposure opening 58 at the side part of the internal flow channel 52. The width D of the internal flow channel 52 is set smaller than an outer diameter φ of the tip end of the insertion cylinder 102. When the insertion cylinder 102 is inserted into the connector 10, the stopper 90 at the side part of the internal flow channel 52 inhibits the insertion of the insertion cylinder 102 due to the relation of D > φ. The stopper 90 also restricts the further elastic deformation of the deformed portion 88, which is elastically deformed due to the insertion of the insertion cylinder 102.

The stopper 90 has a first taper portion 92 that tilts relatively gradually toward the internal flow channel 52 from the edge of the exposure opening 58, and a second taper portion 94 that is formed between the first taper portion 92 and the side wall 52a of the internal flow channel 52 to tilt more sharply than the first taper portion 92. The first and second taper portions 92 and 94 can obliquely receive the deformed portion 88 that is elastically deformed. Especially, the first taper portion 92 tilts to be easy to be along the shape of the bottom surface of the elastically-deformed deformed portion 88, so that the first taper portion 92 can contact the bottom surface of the deformed portion 88 with a wide range.

On the other hand, the second taper portion 94 tilts with a shallow angle relative to the slit 86 that is opened due to the elastic deformation of the deformed portion 88. The second taper portion 94 smoothly guides the second transfusion material supplied from the flow path 104 into the internal flow channel 52. In addition, since the second taper portion 94 is communicated with the expanding portion 55 (guide wall 54), it can smoothly guide the first transfusion material flown into the internal flow channel 52 into the through-hole 76. Specifically, when the first transfusion material is guided upward by the guide wall 54 in a state in which the male connector 100 is not connected to the third port 28, the first transfusion material can be guided along the slope of the first and second taper portions 92 and 94, whereby the first transfusion material can fully be flown into the through-hole 76. Thus, the stay of the first transfusion material in the through-hole 76 can significantly be reduced.

In addition, the side wall 52a of the internal flow channel 52, and the first and second taper portions 92 and 94 are continuously formed to have a smooth curved surface. Therefore, even if the deformed portion 88 is pushed by the insertion cylinder 102, the curved surface can prevent the deformed portion 88 from being forcibly crushed. The stopper 90 formed to have a smooth curved surface can also prevent damage on the deformed portion 88.

The connector 10 according to the present embodiment is basically configured as described above. The operation and effect of the connector 10 will be described below.

The first transfusion material is flown through the connector 10 along the main line flow channel 50 with the first tube 12a being connected to the first port 24 at the upstream side and the second tube 12b being connected to the second port 26 at the downstream side. Accordingly, the internal flow channel 52 and the through-hole 76 of the connector 10 are filled with the first transfusion material flowing through the main line flow channel 50. The male connector 100 of the third tube 12c is inserted into the third port 28 of the connector 10 with this state.

Before the male connector 100 is inserted, the third port 28 is washed with alcohol, for example, to prevent external intrusion of dust or bacteria. In this case, the valve 36 can easily be washed, since the top surface of the valve 36 (upper expanding portion 88a) is formed flat.

As illustrated in Fig. 4, upon the connection of the male connector 100, the tip end of the insertion cylinder 102 is set opposite to the top surface of the valve 36, and is moved toward the valve 36, whereby the insertion cylinder 102 contacts the valve 36. The insertion cylinder 102 is further moved downward (into the connector 10) to push the valve 36 downward. With this pushing motion, the deformed portion 88 is displaced (elastically deformed) downward relative to the cap 32, thereby opening the slit 86.

When the insertion cylinder 102 is inserted downward by a predetermined length, the bottom surface of the deformed portion 88 contacts the stopper 90 as illustrated in Fig. 5. Therefore, the stopper 90 restricts the elastic deformation of the deformed portion 88, and at the same time, restricts the movement of the insertion cylinder 102 that pushes the deformed portion 88 downward. In other words, the deformed portion 88 is held by the stopper 90 and the insertion cylinder 102.

The stopper 90 is configured to stop the movement of the insertion cylinder 102 at the position where the taper surface 102a of the insertion cylinder 102 is fitted to the opening 40 of the cap 32. Therefore, when the insertion cylinder 102 is inserted with an insertion length specified by the stopper 90, the insertion cylinder 102 is tightly bonded to the opening 40 in a liquid-tight manner, whereby the insertion cylinder 102 is easily connected to be held by the connector 10. Further, the excessive insertion of the insertion cylinder 102 into the connector 10 is avoided, whereby the opening 40 can be prevented from being pushed and opened by the taper surface 102a.

The stopper 90 is in the state of obliquely receiving the bottom surface of the deformed portion 88 by the first and second taper portions 92 and 94, so that the stopper 90 contacts the surface of the deformed portion 88 in a wide range. In the through-hole 76 in the third port 28, the elastically deformed valve 36 thoroughly covers around the insertion cylinder 102, so that the tip end and the taper surface 102a of the insertion cylinder 102 contact the surface of the valve 36 so as to receive strong friction force. Accordingly, the insertion cylinder 102 is fixedly held by the third port 28. Even if the insertion cylinder 102 is obliquely inserted into the third port 28, the position of the insertion cylinder 102 can easily be corrected via the valve 36.

In the state in which the deformed portion 88 is sandwiched between the insertion cylinder 102 and the stopper 90, the deformed portion 88 prevents the insertion cylinder 102 from being further inserted, which can surely prevent the insertion cylinder 102 from penetrating through the slit 86. Specifically, the stopper 90 restricts a predetermined or more degree of elastic deformation of the deformed portion 88, thereby being capable of preventing the slit 86 from being opened more than a predetermined degree. This can prevent the insertion cylinder 102, which is inserted into the slit 86, from pushing and opening the slit 86, whereby the trouble of breaking the slit 86 by the deformed portion 88 can significantly be reduced. In addition, since the stopper 90 has a smooth curved surface (the first and second taper portions 92 and 94), the damage on the deformed portion 88 caused by the stopper 90 can be avoided.

The flow path 104 (another line flow channel 110) and the main line flow channel 50 are satisfactorily communicated with each other through the opened slit 86 at the position where the insertion of the insertion cylinder 102 is restricted. When the male connector 100 is removed (moved backward) from the connector 10, the deformed valve 36 is positively and elastically restored to be displaced in the direction in which the male connector 100 is removed (upward). Due to this elastic restoration, the deformed portion 88 enters the opening 40 of the cap 32, and the slit 86 is closed. In this case, since the insertion cylinder 102 does not penetrate through the slit 86, deterioration in the closing property of the slit 86 is prevented, whereby the through-hole 76 is quickly closed, and hence, the leakage of the transfusion material from the connector 10 can be prevented.

As described above, the connector 10 according to the present embodiment includes the stopper 90 on the way where the valve 36 is displaced. Therefore, even if the male connector 100 deforms the valve 36 upon connecting the male connector 100 to the connector 10, the stopper 90 can restrict a predetermined or more degree of elastic deformation of the valve 36. When the elastic deformation of the valve 36 is restricted as described above, the penetration of the male connector 100 through the slit 86 can effectively be prevented. As a result, the connector 10 can significantly reduce the situation in which the valve 36 is broken by the male connector 100 that pushes and opens the slit 86. With this, the connector 10 can satisfactorily be used multiple times or for a long period of time.

In this case, the stopper 90 is formed from the side wall surface of the internal flow channel 52 that is narrower than the outer diameter of the insertion cylinder 102. With this, the elastic deformation of the valve 36 can easily be restricted without separately providing the stopper 90, whereby production cost can be reduced.

Since the stopper 90 is formed continuous with the exposure opening 58 from which the internal flow channel 52 is exposed, the connector 10 can restrict the elastic deformation of the valve 36 around the exposure opening 58. Therefore, the connector 10 can allow the insertion cylinder 102 to be fully inserted to the vicinity of the exposure opening 58 (internal flow channel 52) upon connection, and can satisfactory maintain the connection state with the insertion cylinder 102.

The connector 10 according to the present invention is not limited to the above configuration, and various configurations can obviously be applied. Some modifications of the present invention will be described below. In the description below, the same components as the connector 10 or the components having the similar function to the connector 10 according to the present embodiment are identified by the same reference numerals, and their detailed description will not be repeated.

### First Modification

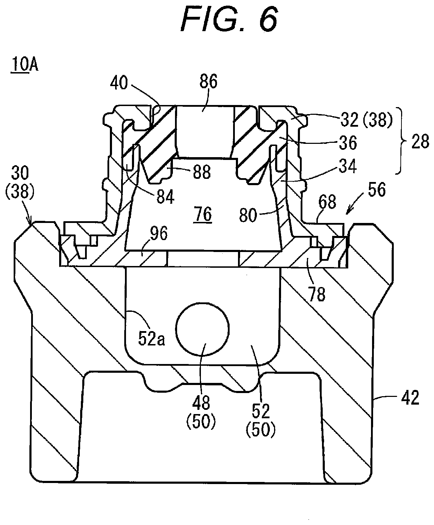

A connector 10A according to a first modification illustrated in Fig. 6 has a configuration in which a stopper 96 is formed on a support member 34 stored in a cap 32. Specifically, the stopper 96 is formed to project to narrow an opening at a lower end of a through-hole 76 in order to restrict a predetermined or more degree of elastic deformation of a valve 36 (deformed portion 88) by its top surface. When the stopper 96 is formed by using a member other than the housing 30 as described above, the effect similar to the effect of the stopper 90 of the connector 10 according to the present embodiment can also be obtained.

As illustrated in Fig. 6, the stopper 96 may be formed such that the surface opposite to the valve 36 is flat, or is formed to have a taper portion as in the stopper 90 according to the present embodiment. In summary, the stoppers 90 and 96 are only provided in the direction in which the valve 36 is displaced to restrict a predetermined or more degree of elastic deformation of the valve 36. The shape and the arrangement position of the stoppers 90 and 96 may appropriately be set depending on the relation with the valve 36.

### Second Modification

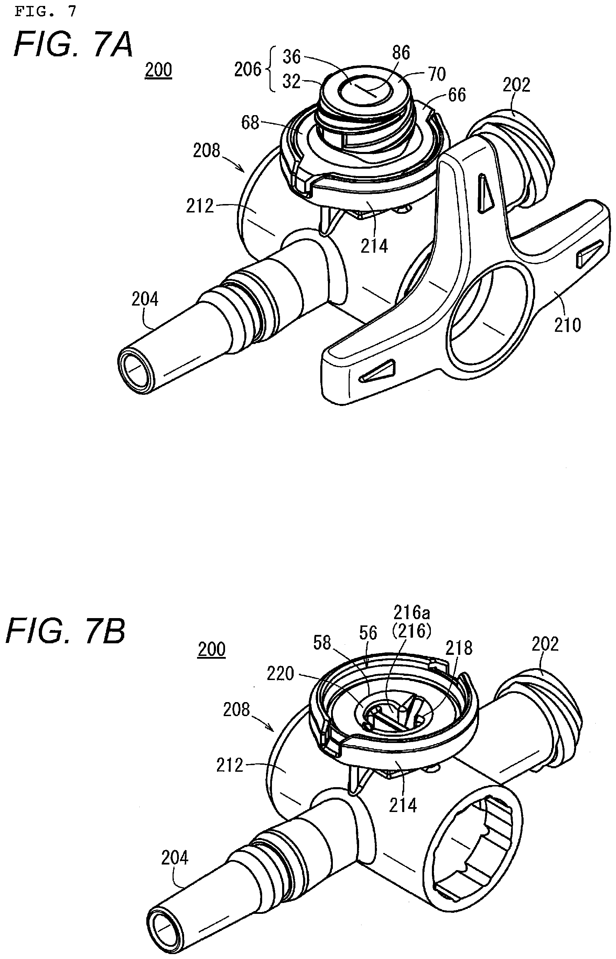

A connector 200 according to a second modification illustrated in Figs. 7A and 7B is configured as a three-way cock including a cock 210 that is rotatably inserted into a body 208 including first to third ports 202 to 206, wherein a flowing state of a transfusion material inside can be changed based on the position where the rotation of the cock 210 stops. A third port support portion 214 extending upward is provided to a cylinder 212 to which the cock 210 is inserted, and a mounting portion 56 similar to the connector 10 according to the present embodiment is formed on the top surface of the third port support portion 214. The third port 206 is mounted to this mounting portion 56. Like the connector 10 according to the present embodiment, the third port 206 includes a cap 32, a support member (not illustrated), and a valve 36. A guide flow channel 216 through which the transfusion material can be flown in the vertical direction is formed in the third port support portion 214.

An exposure opening 58 of the mounting portion 56 has a diameter larger than a wall 216a forming the guide flow channel 216. A stopper 220 with a tapered shape is formed between an edge of the exposure opening 58 and an opening 218 of the guide flow channel 216. The stopper 220 is formed continuous with the wall 216a of the guide flow channel 216, and has a function of restricting a predetermined or more degree of elastic deformation of the valve 36 that is pushed by a male connector (not illustrated). Therefore, the connector 200 configured as a three-way cock according to the second modification can also bring the similar effects to the connector 10 according to the present embodiment.

### Third Modification

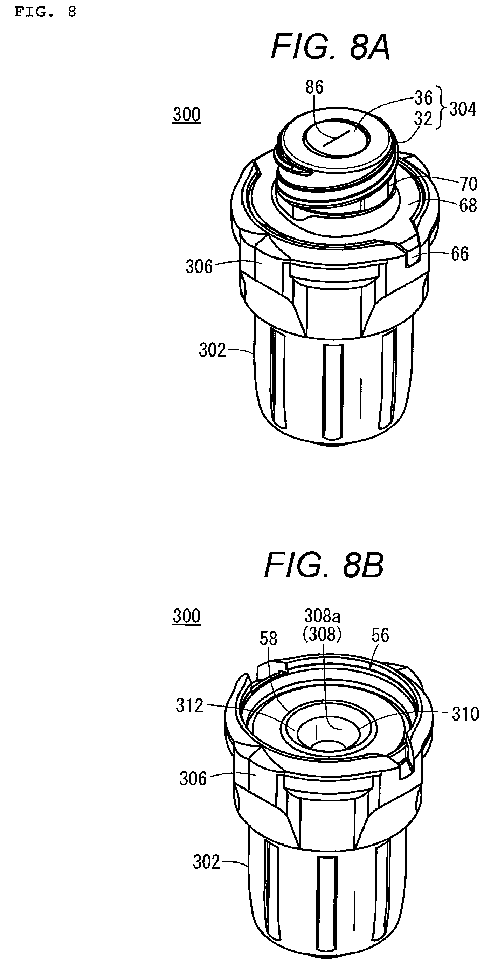

A connector 300 according to a third modification illustrated in Figs. 8A and 8B is configured as an I-port connector having a tip port 302 and a base port 304. This connector 300 is connected to a connection terminal of a CV catheter (central intravenous catheter), for example, and has a function as a protection cap of the connection terminal.

The tip port 302 of the connector 300 is formed as a luer lock male connector. On the other hand, the base port 304 includes a cap 32, a support member (not illustrated), and a valve 36, as in the third port 28 according to the present embodiment. The base port 304 is connected to the connection terminal. A cylindrical intermediate base portion 306 is formed between the tip port 302 and the base port 304, and a flow channel 308 through which a transfusion material can be flown is formed inside to penetrate through the intermediate base portion 306. A mounting portion 56 similar to the connector 10 according to the present embodiment is mounted on the top surface of the intermediate base portion 306, and the base port 304 can be attached to the mounting portion 56.

An exposure opening 58 of the mounting portion 56 is formed to have a diameter larger than a wall 308a forming the flow channel 308. A stopper 312 having a tapered shape is formed between an edge of the exposure opening 58 and an opening 310 of the flow channel 308. The stopper 312 is formed continuous with the wall 308a of the flow channel 308, and has a function of restricting a predetermined or more degree of elastic deformation of the valve 36 that is pushed by the connection terminal. Therefore, the connector 300 configured as an I-port connector according to the third modification can also bring the similar effects to the connector 10 according to the present embodiment.

## Claims

1. A connector (10, 10A, 200, 300) comprising:
a body (38) including a flow channel (50) through which fluid can be circulated, and a space (76) communicating with the flow channel (50); and
a valve (36) including a base (88) disposed in the space (76) so as to be displaceable, and a slit (86) that can open and close the flow channel (50) based on elastic deformation of the base (88), wherein
the body (38) has a stopper (90, 96, 220, 312) provided on a way, on which the valve (36) is displaced, for restricting a predetermined or more degree of elastic deformation of the valve (36),
wherein the stopper (90, 96, 220, 312) holds the elastically deformed base (88) with an inserted body (100) when the inserted body (100) is inserted into the body (38), and
**characterized in that** the stopper (90, 220, 312) is formed to have a taper portion (92, 94) that becomes narrower toward the flow channel (50) from a position where the valve (36) is disposed.

2. The connector (10, 10A, 200, 300) according to claim 1, wherein
the stopper (90, 96, 220, 312) is formed from a wall face at a side part of the flow channel (50) that is narrower than the space (76).

3. The connector (10, 10A, 200, 300) according to claim 2, wherein
the body (38) includes a housing (30) that forms the flow channel (50), and a cover member (32) that is mounted to the housing (30) to form the space (76), and
the stopper (90, 96, 220, 312) is formed to be communicated with an exposure opening (58) of the housing (30) from which the flow channel (50) is exposed.

## Patentansprüche

1. Verbinder (10, 10A, 200, 300), umfassend:
einen Körper (38), der einen Strömungskanal (50) umfasst, durch den Fluid zirkulieren kann, und einen Raum (76), der mit dem Strömungskanal (50) in Verbindung steht; und
ein Ventil (36), das eine Basis (88), die in dem Raum (76) so angeordnet ist, dass sie verschiebbar ist, und einen Schlitz (86) umfasst, der den Strömungskanal (50) basierend auf einer elastischen Verformung der Basis (88) öffnen und schließen kann, wobei
der Körper (38) ein Anschlagelement (90, 96, 220, 312) aufweist, das auf einem Weg vorgesehen ist, auf dem das Ventil (36) verschoben wird, um eine vorbestimmte oder größere elastische Verformung des Ventils (36) zu begrenzen,
wobei das Anschlagelement (90, 96, 220, 312) die elastisch verformte Basis (88) mit einem eingesetzten Körper (100) hält, wenn der eingesetzte Körper (100) in den Körper (38) eingeführt wird, und
**dadurch gekennzeichnet, dass**
das Anschlagelement (90, 220, 312) so ausgebildet ist, dass es einen verjüngten Abschnitt (92, 94) aufweist, der aus einer Position, in der das Ventil (36) angeordnet ist, in Richtung des Strömungskanals (50) schmaler wird.

2. Verbinder (10, 10A, 200, 300) nach Anspruch 1, wobei das Anschlagelement (90, 96, 220, 312) aus einer Wandfläche an einem Seitenteil des Strömungskanals (50) gebildet ist, der schmaler als der Raum (76) ist.

3. Verbinder (10, 10A, 200, 300) nach Anspruch 2, wobei
der Körper (38) ein Gehäuse (30), das den Strömungskanal (50) bildet, und ein Abdeckelement (32) umfasst, das an dem Gehäuse (30) befestigt ist, um den Raum (76) zu bilden, und
das Anschlagelement (90, 96, 220, 312) so ausgebildet ist, dass es mit einer Expositionsöffnung (58) des Gehäuses (30) in Verbindung gebracht werden kann, von der aus der Strömungskanal (50) freigelegt ist.

## Revendications

1. Raccord (10, 10A, 200, 300) comprenant :
un corps (38) comportant un canal d'écoulement (50) dans lequel un fluide peut circuler, et un espace (76) communiquant avec le canal d'écoulement (50) ; et
une valve (36) comportant une base (88) disposée dans l'espace (76) de façon à être déplaçable, et une fente (86) qui peut ouvrir et fermer le canal d'écoulement (50) sur la base d'une déformation élastique de la base (88),
dans lequel le corps (38) présente un arrêtoir (90, 96, 220, 312) situé sur un trajet sur lequel la valve (36) est déplacée, pour limiter un degré prédéterminé ou plus de déformation élastique de la valve (36),
dans lequel l'arrêtoir (90, 96, 220, 312) maintient la base (88) élastiquement déformée avec un corps inséré (100) quand le corps inséré (100) est inséré dans le corps (38), et
**caractérisé en ce que** l'arrêtoir (90, 220, 312) est formé pour présenter une partie conique (92, 94) qui devient plus étroite vers le canal d'écoulement (50) à partir d'une position où la valve (36) est disposée.

2. Raccord (10, 10A, 200, 300) selon la revendication 1, dans lequel
l'arrêtoir (90, 96, 220, 312) est formé à partir d'une face de paroi au niveau d'une partie latérale du canal d'écoulement (50) qui est plus étroite que l'espace (76).

3. Raccord (10, 10A, 200, 300) selon la revendication 2, dans lequel
le corps (38) comporte un boîtier (30) qui forme le canal d'écoulement (50), et un élément de couvercle (32) qui est monté sur le boîtier (30) pour former l'espace (76), et
l'arrêtoir (90, 96, 220, 312) est formé pour être mis en communication avec une ouverture d'exposition (58) du boîtier (30) par laquelle le canal d'écoulement (50) est exposé.
